(19)

**Europäisches Patentamt**
**European Patent Office**
**Office européen des brevets**

(11) **EP 4 659 856 A1**

(12) **EUROPEAN PATENT APPLICATION**
published in accordance with Art. 153(4) EPC

(43) Date of publication:
**10.12.2025  Bulletin 2025/50**

(21) Application number: **24863293.7**

(22) Date of filing: **09.09.2024**

(51) International Patent Classification (IPC):
**B01J 23/745** (2006.01)  **B01J 35/61** (2024.01)
**B01J 35/30** (2024.01)  **B01J 37/03** (2006.01)
**C07C 1/12** (2006.01)

(52) Cooperative Patent Classification (CPC):
**B01J 23/745; B01J 35/30; B01J 35/61;**
**B01J 37/03; C07C 1/12**

(86) International application number:
**PCT/KR2024/013606**

(87) International publication number:
**WO 2025/053714 (13.03.2025 Gazette 2025/11)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority:  **08.09.2023  KR 20230119552**

(71) Applicant: **LG CHEM, LTD.**
**Seoul 07336 (KR)**

(72) Inventors:
  • **LEE, Yong Hee**
    **Daejeon 34122 (KR)**
  • **KIM, Won Hee**
    **Daejeon 34122 (KR)**

  • **JUNG, Da Won**
    **Daejeon 34122 (KR)**
  • **KIM, Seong Min**
    **Daejeon 34122 (KR)**
  • **MOON, Ji Won**
    **Daejeon 34122 (KR)**
  • **PARK, Seung Won**
    **Daejeon 34122 (KR)**
  • **AN, Kwang Jin**
    **Ulju-gun Ulsan 44919 (KR)**
  • **LEE, Ho Jeong**
    **Ulju-gun Ulsan 44919 (KR)**
  • **HUR, Chang Hun**
    **Ulju-gun Ulsan 44919 (KR)**

(74) Representative: **Hoffmann Eitle**
**Patent- und Rechtsanwälte PartmbB**
**Arabellastraße 30**
**81925 München (DE)**

(54) **CATALYST FOR DIRECT HYDROGENATION OF CARBON DIOXIDE, METHOD FOR PRODUCING SAME, AND METHOD FOR PREPARING HYDROCARBON COMPOUND USING SAME**

(57)  The present invention relates to a catalyst useful for a direct hydrogenation reaction of carbon dioxide, a method for producing same, and a method for preparing a hydrocarbon compound using same, and provides a metal-oxide-based catalyst comprising an oxide-based support and catalyst particles containing iron supported on the support, a method for producing same, and a method for preparing a hydrocarbon compound using same.

EP 4 659 856 A1

Description

**[Technical Field]**

[CROSS-REFERENCE TO RELATED APPLICATION]

This application claims the benefit of priority to Korean Patent Application No. 10-2023-0119552 filed on September 8, 2023, the disclosure of which is incorporated herein by reference in its entirety.

[Technical Field]

The present invention relates to a catalyst useful for carbon dioxide direct hydrogenation, a method for preparing thereof, and a method for preparing hydrocarbon compounds through carbon dioxide direct hydrogenation using the same.

**[Background Art]**

Coal and petroleum are fossil fuels that account for 50% or more of total energy and have been used as important energy sources for mankind for the past several centuries. Mankind has been emitting thermodynamically stable carbon dioxide without a separate post-treatment process while going through various energy conversion processes thereof.
However, as it has recently become known that carbon dioxide is the main greenhouse gas that contributes 55% to global warming, various technologies for removing carbon dioxide have been proposed. Among them, technologies that use catalysts to hydrogenate carbon dioxide and convert it into high value-added chemical fuels such as alpha olefins or liquid fuels such as gasoline and diesel are most preferred because they are easy to technically link with existing industrial processes, produce products that have secured a large markets and can easily process large amounts of carbon dioxide.
Carbon dioxide conversion technology is a technology that converts carbon dioxide, which is the most stable among carbon compounds, into other useful compounds, including, for example, a technology that converts carbon dioxide into carbon monoxide, methanol, and hydrocarbon compounds through carbon dioxide hydrogenation.
The hydrogenation reaction of carbon dioxide generally comprises a first step in which carbon dioxide supplied as a reactant is converted into carbon monoxide through a reverse water gas shift (RWGS) reaction, and a second step in which the generated carbon monoxide is combined with hydrogen through a Fischer-Tropsch synthesis (FTS) reaction to be converted into hydrocarbons.
Here, the reverse water gas shift reaction of the first step is an endothermic reaction and may be expressed by Equation 1 below:

$$[\text{Equation 1}] \qquad CO_2 + H_2 \rightarrow CO + H_2O \quad \Delta_R H^0{}_{300} = 38KJ/mol$$

The reverse water gas shift reaction of the first step is a reversible reaction and can be performed under conditions that provide partial conversion of carbon dioxide and hydrogen.
The carbon monoxide produced in the first step undergoes the Fischer-Tropsch synthesis (FTS) reaction of the second stage, wherein the Fischer-Tropsch synthesis reaction is exothermic and may be expressed by Equation 2 below:

[Equation 2]

1) When the product is a paraffin: $nCO + (2n+1)H_2 \rightarrow C_nH_{2n+2} + nH_2O$
2) When the product is an olefin: $nCO + 2nH_2 \rightarrow C_nH_{2n} + nH_2O$

The reverse water gas shift reaction in the first step and the Fischer-Tropsch synthesis reaction in the second step are each configured as separate processes and are sometimes connected to each other, but the reverse water gas shift reaction in the first step is an endothermic reaction that requires a high reaction temperature to secure a high conversion rate, so there is a problem that the energy efficiency of the entire conversion process is reduced. Accordingly, research is being conducted on a carbon dioxide direct hydrogenation reaction in which the reverse water gas shift reaction in the first step and the Fischer-Tropsch synthesis reaction in the second step can be performed simultaneously in a single catalyst and process.
This carbon dioxide direct hydrogenation reaction in which the reverse water gas shift reaction and the Fischer-Tropsch synthesis reaction are performed simultaneously in a single catalyst and process is an exothermic reaction and may be expressed by Equation 3 below:

[Equation 3]

1) When the product is a paraffin: $nCO_2+(3n+1)H_2 \rightarrow C_nH_{2n+2}+2nH_2O$
2) When the product is an olefin: $nCO_2+3nH_2 \rightarrow C_nH_{2n}+2nH_2O$

In particular, when the heat of reaction accumulates inside the catalyst particle during the Fischer-Tropsch synthesis reaction, the selectivity for the hydrocarbon to be synthesized decreases and the catalyst deteriorates, so it is very important to quickly remove the heat of reaction from the catalyst particle. Accordingly, there has been performed a method of appropriately controlling the reactivity by including a certain amount of inert support when forming a fixed-bed catalyst layer or by mixing any inert particles with the catalyst particles to form a catalyst layer.

However, when the inert support is used, since the support itself is a porous material, there is a problem that the catalyst material permeates inside the support and causes a catalytic reaction to occur inside, making it difficult to control the exothermic reaction inside. When using the inert particles by physically mixing them with the catalyst particles, there is a problem that uniform mixing is difficult, and the catalyst material partially clumps up, causing the reaction to occur unevenly.

In addition, slurry reactions such as fluidized bed reactions are effective in terms of heat control and reaction efficiency, but in this case, it is necessary to secure the mechanical properties of the catalyst, and thus, a method of adding a structural promoter to the catalyst has been performed. However, when the structural promoter, which is inactive, is added, there is a problem that the content of the active component decreases, and the carbon dioxide conversion rate is severely reduced due to the decrease in the active ingredient.

**[Prior Art Literature]**

**[Patent Documents]**

(Patent Document 1) KR 10-1405090 B1 (2014.06.02)

**[Disclosure]**

**[Technical Problem]**

The present invention is intended to solve the above problems, and its object is to provide a catalyst useful for direct hydrogenation of carbon dioxide, which has high reaction activity by including a high content of catalytically active components, and also excellent specific surface area and mechanical properties by including a structural promoter component.

Another object of the present invention is to provide a method for preparing the catalyst.

In addition, the present invention aims to provide a method for producing a hydrocarbon compound, including a step of direct hydrogenation of carbon dioxide in the presence of the catalyst.

**[Technical Solution]**

In order to solve the above problem, the present invention provides a catalyst, a method for preparing the same, and a method for producing a hydrocarbon compound.

(1) The present invention provides a metal oxide-based catalyst, comprising: an oxide-based carrier; and catalyst particles including iron supported on the carrier, wherein the content of iron per 1 $m^2$ of surface area of the catalyst is 5 mg to 15 mg.

(2) The present invention provides the catalyst according to (1) above, wherein the iron is included in an amount of 50 wt% or more and 65 wt% or less based on 100 wt% of the total catalyst.

(3) The present invention provides the catalyst according to (1) or (2) above, wherein the specific surface area of the catalyst is 50 $m^2$/g to 100 $m^2$/g.

(4) The present invention provides the catalyst according to any one of (1) to (3) above, wherein the oxide-based carrier is at least one selected from the group consisting of $SiO_2$, $Al_2O_3$, and zeolite.

(5) The present invention provides the catalyst according to any one of (1) to (4) above, wherein the iron is in a hematite phase.

(6) The present invention provides the catalyst according to any one of (1) to (5) above, wherein the iron is activated to at least one selected from the group consisting of magnetite, iron carbide, and metallic iron (Fe).

(7) The present invention provides the catalyst according to any one of (1) to (6) above, wherein the catalyst particles further include a promoter metal, wherein the promoter metal is at least one selected from the group consisting of copper, potassium, and sodium.

(8) The present invention provides the catalyst according to any one of (1) to (7) above, wherein the catalyst particles further include at least one promoter metal selected from the group consisting of copper, potassium, and sodium, wherein the promoter metal is included in an amount of 0.1 to 20 parts by weight based on 100 parts by weight of the catalyst.

(9) The present invention provides the catalyst according to any one of (1) to (8) above, wherein the catalyst is a catalyst for direct hydrogenation of carbon dioxide.

(10) The present invention provides a method for preparing the catalyst according to any one of (1) to (9) above, the method including the steps of: preparing iron oxide-based catalyst particles (S1); supporting a promoter metal on the catalyst particles (S2); and drying and calcining (S3), wherein the step (S1) comprises the steps of: preparing a first solution by dissolving an iron precursor in a solvent to have an iron weight concentration of 20 g/L to 100 g/L, and introducing and dispersing an oxide-based carrier into the first solution to prepare a second solution (S1-1); stirring the second solution to raise the temperature to 40°C to 90°C, and adding an aqueous precipitant solution to prepare a precipitate including iron oxide-based catalyst particles (S1-2); and washing, filtering, and then drying the precipitate (S1-3), wherein in step (S1-1), the oxide-based carrier is introduced in an amount such that the iron content per 1 $m^2$ of the oxide-based carrier surface area is 10 mg to 150 mg, and wherein in step (S2), the promoter metal is supported in an amount such that the weight ratio of iron/promoter metal is 5 to 15.

(11) The present invention provides the method for preparing a catalyst according to (10) above, wherein in step (S1-2) above, the aqueous precipitant solution contains a precipitant at a weight concentration of 50 g/L to 250 g/L, wherein the precipitant is at least one selected from the group consisting of sodium carbonate, ammonium carbonate, ammonia water, and sodium hydroxide.

(12) The present invention provides the method for preparing a catalyst according to (10) or (11) above, wherein in step (S1-2) above, the aqueous precipitant solution is added until the pH in the reactor becomes 7 to 8.

(13) The present invention provides the method for preparing a catalyst according to any one of (10) to (12) above, wherein the iron precursor is at least one selected from the group consisting of iron nitrate, sulfate, acetate, chloride, bromide, iodide, and hydrates thereof.

(14) The present invention provides the method for preparing a catalyst according to any one of (10) to (13) above, wherein in step (S1-2) above, the stirring is performed at a speed of 100 rpm to 1000 rpm.

(15) The present invention provides the method for preparing a catalyst according to any one of (10) to (14) above, wherein in step (S1-2) above, after the addition of the aqueous precipitant solution is completed, further stirring is performed for 0.5 to 24 hours.

(16) The present invention provides the method for preparing a catalyst according to any one of (10) to (15) above, wherein in step (S2) above, the promoter metal is at least one selected from the group consisting of copper, potassium, and sodium.

(17) The present invention provides the method for preparing a catalyst according to any one of (10) to (16) above, wherein step (S2) above is performed by an incipient wetness impregnation method.

(18) The present invention provides the method for preparing a catalyst according to any one of (10) to (17) above, wherein in steps (S1-3) and (S3) above, the drying is performed at 100°C to 200°C for 5 to 24 hours, respectively.

(19) The present invention provides the method for preparing a catalyst according to any one of (10) to (18) above, wherein in step (S3) above, the calcination is performed by heat treatment at a temperature of 200°C to 600°C for 1 to 12 hours.

(20) The present invention provides a method for producing a hydrocarbon compound, including a step of direct hydrogenation of carbon dioxide in the presence of the catalyst described in any one of (1) to (9) above.

[Advantageous Effects]

The catalyst according to the present invention includes a high content of iron as a catalyst component and has a specific surface area and iron content per catalyst surface area within a specific range, thereby exhibiting the effects of having high catalytic reaction activity, excellent carbon dioxide conversion rate and turnover frequency (TOF), and excellent selectivity for high value-added C5 or higher hydrocarbon compounds.

In addition, the method for preparing a catalyst according to the present invention controls the ratio of an oxide-based carrier to an iron precursor to support iron on the carrier, dry it, and then support a promoter metal thereon at a controlled ratio, thereby enabling the production of a catalyst having a specific surface area and iron content per surface area in a specific range while including iron in a specific range.

Furthermore, the method for producing a hydrocarbon according to the present invention uses the above catalyst, and thus has excellent carbon dioxide conversion rate and TOF while also having high selectivity for hydrocarbon compounds of C5 or higher, so that it can be more useful for the production of hydrocarbon compounds of C5 or higher.

**[Best Modes of the Invention]**

The terms or words used in the specification and claims of the present application should not be construed as being limited to their ordinary or dictionary meanings, but should be interpreted as meanings and concepts consistent with the technical spirit of the present invention, based on the principle that the inventor may adequately define the concepts of terms to best describe his invention.

The present invention provides a catalyst useful for a carbon dioxide direct hydrogenation reaction, particularly a Fischer-Tropsch synthesis reaction, a method for preparing the same, and a method for producing a hydrocarbon compound using the same.

Hereinafter, the present invention will be described in more detail.

Catalyst

The present invention provides a catalyst having excellent catalytic activity while also having excellent specific surface area and mechanical properties by being supported on a carrier.

The catalyst according to one embodiment of the present invention is characterized by being a metal oxide-based catalyst comprising: an oxide-based carrier; and catalyst particles including iron supported on the carrier, wherein the content of iron per 1 $m^2$ of surface area of the catalyst is 5 mg to 15 mg.

In addition, the catalyst may include 50 wt% or more and 65 wt% or less of the iron based on 100 wt% of the total catalyst, and may have a specific surface area of 50 $m^2/g$ to 100 $m^2/g$.

The hydrogenation reaction of carbon dioxide generally comprises a first step in which carbon dioxide supplied as a reactant is converted into carbon monoxide through a reverse water gas shift (RWGS) reaction, and a second step in which the generated carbon monoxide is combined with hydrogen through a Fischer-Tropsch synthesis (FTS) reaction to be converted into hydrocarbons.

In particular, in order to control the reaction heat and improve the mechanical properties inside the catalyst particle during the Fischer-Tropsch synthesis reaction, a method of adding a structural promoter (support) to the catalyst has been performed. However, when the structural promoter, which is inactive, is added, there is a problem that the content of the catalytically active component decreases, and the carbon dioxide conversion rate is severely reduced due to the decrease in the active ingredient.

In addition, when the reverse water gas shift reaction in the first step and the Fischer-Tropsch synthesis reaction in the second step are each configured as separate processes and are performed sequentially, the reverse water gas shift reaction in the first step is an endothermic reaction that requires a high reaction temperature to secure a high conversion rate, so there is a problem that the energy efficiency of the entire conversion process is reduced. Accordingly, research is being conducted on a carbon dioxide direct hydrogenation reaction in which the reverse water gas shift reaction in the first step and the Fischer-Tropsch synthesis reaction in the second step can be performed simultaneously in a single catalyst and process, and the development of such a catalyst is necessary.

The catalyst according to one embodiment of the present invention includes a high content of iron in the form of a catalyst particle supported on an oxide-based carrier, which is a structural promoter, and has a specific surface area and iron content per surface area within a specific range, thereby exhibiting the effects of having high catalytic reaction activity, excellent carbon dioxide conversion rate and turnover frequency (TOF), and excellent selectivity for high value-added C5 or higher hydrocarbon compounds, in the Fischer-Tropsch synthesis reaction and in the process of simultaneously performing the reverse water gas shift reaction and the Fischer-Tropsch synthesis reaction.

The catalyst according to one embodiment of the present invention is a metal oxide-based supported catalyst, which comprises an oxide-based carrier, and catalyst particles supported on the carrier, wherein the catalyst particles include iron. In addition, the iron may be in a hematite phase.

In addition, the catalyst is activated through pretreatment and then used in a direct hydrogenation reaction of carbon dioxide, wherein the iron may be activated to at least one selected from the group consisting of magnetite, iron carbide, and metallic iron (Fe).

As another example, the iron in the catalyst may be included in the catalyst in a hematite phase and then activated to one or more selected from the group consisting of magnetite, iron carbide, and metallic iron (Fe) through pretreatment.

In addition, the catalyst may include 50 wt% or more and 65 wt% or less of the iron based on 100 wt% of the total catalyst, and may have a specific surface area of 50 $m^2/g$ to 100 $m^2/g$, and an iron content of 5 mg to 15 mg per 1 $m^2$ of the catalyst surface area. Specifically, the catalyst may include the iron in an amount of 55 wt% to 60 wt% based on 100 wt% of the total catalyst, and may have a specific surface area of 52 m$^2$/g to 70 m$^2$/g, and an iron content of 8 mg to 12 mg per 1 m$^2$ of the catalyst surface area. In this case, when a hydrocarbon compound is produced through the direct hydrogenation of carbon dioxide using the catalyst, the catalytically active component can be sufficient to provide excellent catalytic reaction activity, and further the selectivity for hydrocarbon compounds of C5 or higher and the carbon dioxide conversion rate can be excellent.

The oxide-based carrier is a structural promoter that plays a role in improving the mechanical strength and specific surface area of the catalyst, and specifically, may be at least one selected from the group consisting of SiO$_2$, Al$_2$O$_3$, and zeolite.

In addition, the oxide-based carrier may be SiO$_2$ or Al$_2$O; in consideration of not adversely affecting the performance and physicochemical properties of the catalyst due to the improvement of the mechanical strength and specific surface area of the catalyst and the interaction by the bonding force with the catalyst component.

The catalyst particles may further include a promoter metal as needed, wherein the promoter metal may be at least one selected from the group consisting of copper, potassium, and sodium.

In addition, the catalyst particles may further include at least one selected from the group consisting of copper, potassium, and sodium, and may include the promoter metal in an amount of 0.1 to 20 parts by weight, specifically 1 to 20 parts by weight, based on 100 parts by weight of the catalyst.

In addition, when two or more promoter metals are included, the two or more promoter metals may be included in the same amount.

If the catalyst particles further include copper, the reducing power of the catalyst can be further increased, and if the catalyst particles further include potassium, the basicity of the catalyst surface can be increased, and the selectivity for hydrocarbon compounds of C5 or higher can be more advantageously improved.

## Method of preparing catalysts

The present invention provides a method for preparing the catalyst.

The method for preparing the catalyst according to an embodiment of the present invention is characterized by including the steps of: preparing iron oxide-based catalyst particles (S1); supporting a promoter metal on the catalyst particles (S2); and drying and calcining (S3), wherein the step (S1) comprises the steps of: preparing a first solution by dissolving an iron precursor in a solvent to have an iron weight concentration of 20 g/L to 100 g/L, and introducing and dispersing an oxide-based carrier into the first solution to prepare a second solution (S1-1); stirring the second solution to raise the temperature to 40°C to 90°C, and adding an aqueous precipitant solution to prepare a precipitate including iron oxide-based catalyst particles (S1-2); and washing, filtering, and then drying the precipitate (S1-3), wherein in step (S1-1), the oxide-based carrier is introduced in an amount such that the iron content per 1 m$^2$ of the oxide-based carrier surface area is 10 mg to 150 mg, and wherein in step (S2), the promoter metal is supported in an amount such that the weight ratio of iron/promoter metal is 5 to 15.

Hereinafter, the method for preparing the catalyst will be described in more detail by dividing it into steps.

### *Step (S1)*

Step (S1) is a step of preparing iron oxide-based catalyst particles in which iron, which is a catalytically active component, is supported on an oxide-based carrier, and may be performed by the steps of: preparing a first solution by dissolving an iron precursor in a solvent to have an iron weight concentration of 20 g/L to 100 g/L, and introducing and dispersing an oxide-based carrier into the first solution to prepare a second solution (S1-1); stirring the second solution to raise the temperature to 40°C to 90°C, and adding an aqueous precipitant solution to prepare a precipitate including iron oxide-based catalyst particles (S1-2); and washing, filtering, and then drying the precipitate (S1-3).

The above step (S1-1) is a preliminary step for producing a precipitate, which is an iron oxide-based catalyst cake, by performing a co-precipitation reaction between an iron precursor and an oxide-based carrier to support iron, which is a catalytically active component derived from the iron precursor, on the carrier, and is performed by dissolving an iron precursor in a solvent to prepare a first solution having an iron weight concentration of 20 g/L to 100 g/L, and introducing and dispersing an oxide-based carrier into the first solution to prepare a second solution.

Specifically, the first solution is a solution in which an iron precursor is dissolved in a solvent, wherein the iron precursor may be anyone widely used in the art without particular limitation, and may be, for example, at least one selected from the group consisting of iron nitrate, sulfate, acetate, chloride, bromide, iodide, and hydrates thereof. Specifically, the iron precursor may be at least one selected from iron nitrate, iron chloride, iron sulfate, iron acetate, and hydrates thereof.

In addition, the solvent used in the first solution may be at least one selected from distilled water, ethanol, methanol, and acetone, and specifically, may be distilled water.

In addition, the second solution is prepared by adding and dispersing an oxide-based carrier to the first solution, wherein the oxide-based carrier may be at least one selected from the group consisting of SiO$_2$, Al$_2$O$_3$, and zeolite.

In addition, when preparing the second solution, the oxide-based carrier may be added in an amount such that the iron content per 1 $m^2$ of the oxide-based carrier surface area is 10 mg to 150 mg. Specifically, the oxide-based carrier may be added in an amount such that the iron content per 1 $m^2$ of the oxide-based carrier surface area is 15 mg to 130 mg, 20 mg to 100 mg, or 25 mg to 80 mg.

The above step (S1-2) is a step for preparing a precipitate, which is an iron oxide-based catalyst cake, by performing a co-precipitation reaction between an iron precursor and an oxide-based carrier to support iron, which is a catalytically active component derived from the iron precursor, on the carrier, and may be performed by raising the temperature of the second solution to 40°C to 90°C while stirring the second solution, and adding an aqueous precipitant solution, wherein the stirring may be performed at a speed of 100 rpm to 1000 rpm. If the aqueous precipitant solution is added while stirring under the above conditions, the oxide-based carrier can be uniformly dispersed in the solution, so that iron, which is a catalytically active component, can be evenly precipitated on the surface of the oxide-based carrier, thereby obtaining iron oxide-based catalyst particles having a uniform composition.

In addition, the aqueous precipitant solution may be added until the pH in the reactor becomes 7 to 8, and further stirring may be performed for 0.5 to 24 hours after the addition is completed, wherein the stirring may be performed while maintaining the stirring speed and temperature when the aqueous precipitant solution is added.

As another example, the above step (S1-2) may be performed by raising the temperature of the second solution in the reactor to 40°C to 90°C while stirring the second solution at a speed of 100 rpm to 1000 rpm, adding an aqueous precipitant solution until the pH in the reactor becomes 7 to 8, and further stirring for 0.5 to 24 hours while maintaining the speed and temperature after the addition is completed.

The aqueous precipitant solution may contain the precipitant at a weight concentration of 50 g/L to 250 g/L, wherein the precipitant is at least one selected from the group consisting of sodium carbonate, ammonium carbonate, ammonia water, and sodium hydroxide.

The above step (S1-2) is a step for separating and obtaining iron oxide-based catalyst particles from the produced precipitate, and may be performed by filtering, washing, and drying the precipitate.

The filtering and washing may be performed according to a method widely known in the art, and the drying may be performed so that the precipitate is completely dried, for example, at 100°C to 200°C for 5 to 24 hours.

*Step (S2)*

Step (S2) is a step for further supporting a promoter metal component, and may be performed by supporting a promoter metal on the iron oxide-based catalyst particles manufactured in (S1) step above, wherein the promoter metal may be supported in an amount such that the weight ratio of iron/promoter metal is 5 to 15. In addition, the promoter metal may be at least one selected from the group consisting of copper, potassium, and sodium, wherein the supporting of the promoter metal in step (S2) may be performed by an incipient wetness impregnation method.

Here, the incipient wetness impregnation method may be performed as is widely known in the art, and for example, may be performed by sequentially performing the steps of: crushing iron oxide-based catalyst particles to obtain powder and measuring the pore volume; adding and mixing an aqueous promoter metal precursor solution to the powder; and drying it. Here, the aqueous promoter metal precursor solution may be added in an amount equal to the pore volume or an integer multiple volume thereof, and when added in the integer multiple volume, the addition and drying may be performed repeatedly as many times as the integer multiple.

The aqueous promoter metal precursor solution is a solution in which a promoter metal precursor is dissolved in distilled water, wherein the promoter metal precursor may be at least one selected from the group consisting of nitrates, sulfates, acetates, chlorides, bromides, iodides, and their hydrates of the promoter metal.

Specifically, when the promoter metal is copper, the copper precursor may be at least one selected from the group consisting of copper nitrate, copper chloride, copper acetate, copper sulfate, and hydrates thereof, and more specifically, may be copper nitrate hydrate.

In addition, when the promoter metal is potassium, the potassium precursor may be at least one selected from the group consisting of potassium nitrate, potassium chloride, potassium carbonate, potassium acetate, and potassium sulfate, and more specifically, may be potassium nitrate.

In addition, when the promoter metal is sodium, the sodium precursor may be at least one selected from the group consisting of sodium nitrate, sodium chloride, sodium carbonate, sodium acetate, and sodium sulfate, and more specifically, may be sodium carbonate.

*Step (S3)*

Step (S3) is a step for preparing a catalyst in which the catalyst particles are formed in the form of an oxide through heat treatment, and may be performed by drying and calcination, wherein the drying may be performed at 100°C to 200°C for 5 to 24 hours, and the calcination may be performed by heat treatment at a temperature of 200°C to 600°C for 1 to 12 hours. If the drying and calcination are performed under the above conditions, the iron component in the catalyst can be completely oxidized to obtain a catalyst in the form of hematite, and impurities such as moisture, nitrogen oxides, and chloride remaining in the catalyst can be removed.

**Method for producing hydrocarbon compound**

The present invention provides a method for producing a hydrocarbon compound from carbon dioxide using the catalyst.

According to one embodiment of the present invention, the method for producing a hydrocarbon compound includes a step of directly hydrogenating carbon dioxide in the presence of the catalyst.

Here, the direct hydrogenation of carbon dioxide may be performed in the same manner as a method widely known in the art, except that the above-mentioned catalyst is used.

Specifically, the method for producing a hydrocarbon compound includes the steps of: loading a catalyst into a reactor and performing a reduction treatment under a hydrogen atmosphere; and injecting raw material gas and directly hydrogenating carbon dioxide, wherein the direct hydrogenation of carbon dioxide includes a reverse water gas shift (RWGS) reaction and a Fischer-Tropsch synthesis reaction (FTS).

The above reduction treatment may be carried out by flowing hydrogen through the catalyst at 20 mL/g-catalyst/min to 100 mL/g-catalyst/min at 1 atm to 10 atm and 300°C to 500°C, and the above carbon dioxide direct hydrogenation reaction may be carried out at 200°C to 500°C, a reaction pressure of 1 atm to 100 atm, and a space velocity of 100 $h^{-1}$ to 20,000 $h^{-1}$.

In addition, the above reaction may be carried out without limitation in all reactors widely used in the art, such as a gaseous fixed bed reactor, a fluidized bed reactor, and a liquid slurry type reactor.

**EXAMPLES**

Hereinafter, examples of the present invention will be described in detail so that those skilled in the art can easily implement the present invention. However, the present invention may be embodied in a variety of forms and is not limited to the examples described herein.

**Example 1**

0.36 g of porous silica powder having a specific surface area of 395 $m^2$/g and 200 mL of a solution of iron nitrate hydrate (55.8 g/L) dissolved in distilled water were added into a precipitation reaction vessel, and the temperature was raised to 60°C while stirring at 400 rpm. In this case, the porous silica powder was input in an amount such that the iron content per 1 $m^2$ of its surface area was 78 mg. After the temperature of the solution reached 60°C, 180 g/L of aqueous ammonium carbonate solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.4 after the precipitation was completed. The produced precipitate was filtered to be separated from the solvent, washed with 2.5 L of distilled water, and dried at 110°C for 12 hours (completely dried).

The content of iron in the Fe-SiO$_2$ powder obtained after drying was 60 wt%, and the content of iron was adjusted by adding copper and potassium. Copper and potassium were supported by adding an aqueous solution containing 153 g/L of copper nitrate hydrate and 153 g/L of potassium nitrate to the Fe-SiO$_2$ powder so that the weight ratio of iron/(copper + potassium) was 6. In this case, the copper and potassium were supported in the same weight. After the addition of copper and potassium, drying was performed at 110°C for 12 hours and calcination was performed at 400°C for 3 hours to manufacture a catalyst.

**Example 2**

2.23 g of porous alumina ($\gamma$-Al$_2$O$_3$) powder having a specific surface area of 200 $m^2$/g and 200 mL of a solution of iron nitrate hydrate (55.8 g/L) dissolved in distilled water were added into a precipitation reaction vessel, and the temperature was raised to 60°C while stirring at 400 rpm. In this case, the porous alumina powder was input in an amount such that the iron content per 1 $m^2$ of its surface area was 25 mg. After the temperature of the solution reached 60°C, 180 g/L of aqueous ammonium carbonate solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.3 after the precipitation was completed. The produced precipitate was filtered to be separated from the solvent, washed with 2.5 L of distilled water, and dried at 110°C for 12 hours (completely dried).

The content of iron in the Fe-Al$_2$O$_3$ powder obtained after drying was 60 wt%, and the content of iron was adjusted by adding copper and potassium. Copper and potassium were supported by adding an aqueous solution containing 62 g/L of copper nitrate hydrate and 62 g/L of potassium nitrate to the Fe-Al$_2$O$_3$ powder so that the weight ratio of iron/(copper + potassium) was 10. In this case, the copper and potassium were supported in the same weight. After the addition of copper and potassium, drying was performed at 110°C for 12 hours and calcination was performed at 400°C for 3 hours to manufacture a catalyst.

**Example 3**

1.84 g of porous silica powder having a specific surface area of 395 m$^2$/g and 200 mL of a solution of iron nitrate hydrate (55.8 g/L) dissolved in distilled water were added into a precipitation reaction vessel, and the temperature was raised to 60°C while stirring at 400 rpm. In this case, the porous silica powder was input in an amount such that the iron content per 1 m$^2$ of its surface area was 15 mg. After the temperature of the solution reached 60°C, 180 g/L of aqueous ammonium carbonate solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.3 after the precipitation was completed. The produced precipitate was filtered to be separated from the solvent, washed with 2.5 L of distilled water, and dried at 110°C for 12 hours (completely dried).

The content of iron in the Fe-SiO$_2$ powder obtained after drying was 63 wt%, and the content of iron was adjusted by adding copper and potassium. Copper and potassium were supported by adding an aqueous solution containing 153 g/L of copper nitrate hydrate and 153 g/L of potassium nitrate to the Fe-SiO$_2$ powder so that the weight ratio of iron/(copper + potassium) was 5.5. In this case, the copper and potassium were supported in the same weight. After the addition of copper and potassium, drying was performed at 110°C for 12 hours and calcination was performed at 400°C for 3 hours to manufacture a catalyst.

**Example 4**

0.23 g of porous silica powder having a specific surface area of 395 m$^2$/g and 200 mL of a solution of iron nitrate hydrate (55.8 g/L) dissolved in distilled water were added into a precipitation reaction vessel, and the temperature was raised to 60°C while stirring at 400 rpm. In this case, the porous silica powder was input in an amount such that the iron content per 1 m$^2$ of its surface area was 121 mg. After the temperature of the solution reached 60°C, 180 g/L of aqueous ammonium carbonate solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.1 after the precipitation was completed. The produced precipitate was filtered to be separated from the solvent, washed with 2.5 L of distilled water, and dried at 110°C for 12 hours (completely dried).

The content of iron in the Fe-SiO$_2$ powder obtained after drying was 69 wt%, and the content of iron was adjusted by adding copper and potassium. Copper and potassium were supported by adding an aqueous solution containing 15 g/L of copper nitrate hydrate and 15 g/L of potassium nitrate to the Fe-SiO$_2$ powder so that the weight ratio of iron/(copper + potassium) was 13. In this case, the copper and potassium were supported in the same weight. After the addition of copper and potassium, drying was performed at 110°C for 12 hours and calcination was performed at 400°C for 3 hours to manufacture a catalyst.

**Example 5**

0.41 g of porous silica powder having a specific surface area of 395 m$^2$/g and 200 mL of a solution of iron nitrate hydrate (55.8 g/L) dissolved in distilled water were added into a precipitation reaction vessel, and the temperature was raised to 60°C while stirring at 400 rpm. In this case, the porous silica powder was input in an amount such that the iron content per 1 m$^2$ of its surface area was 68 mg. After the temperature of the solution reached 60°C, 180 g/L of aqueous ammonium carbonate solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.2 after the precipitation was completed. The produced precipitate was filtered to be separated from the solvent, washed with 2.5 L of distilled water, and dried at 110°C for 12 hours (completely dried).

The content of iron in the Fe-SiO$_2$ powder obtained after drying was 68 wt%, and the content of iron was adjusted by adding copper and potassium. Copper and potassium were supported by adding an aqueous solution containing 50 g/L of copper nitrate hydrate and 50 g/L of potassium nitrate to the Fe-SiO$_2$ powder so that the weight ratio of iron/(copper + potassium) was 12. In this case, the copper and potassium were supported in the same weight. After the addition of copper and potassium, drying was performed at 110°C for 12 hours and calcination was performed at 400°C for 3 hours to manufacture a catalyst.

**Comparative Example 1**

10.4 g of porous silica powder having a specific surface area of 395 $m^2$/g and 200 mL of a solution of iron nitrate hydrate (27.9 g/L) dissolved in distilled water were added into a precipitation reaction vessel, and the temperature was raised to 60°C while stirring at 400 rpm. In this case, the porous silica powder was input in an amount such that the iron content per 1 $m^2$ of its surface area was 1.5 mg. After the temperature of the solution reached 60°C, 72 g/L of aqueous ammonium carbonate solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.5 after the precipitation was completed. The produced precipitate was filtered to be separated from the solvent, washed with 2.5 L of distilled water, and completely dried at 110°C for 12 hours or more.

The content of iron in the Fe-$SiO_2$ powder obtained after drying was 30 wt%, and the content of iron was adjusted by adding potassium. Potassium was supported by adding an aqueous solution containing 30 g/L of potassium carbonate to the Fe-$SiO_2$ powder so that the weight ratio of iron/potassium was 16. After the addition of potassium, drying was performed at 110°C for 12 hours and calcination was performed at 400°C for 3 hours to manufacture a catalyst.

**Comparative Example 2**

2.23 g of porous silica powder having a specific surface area of 395 $m^2$/g and 200 mL of a solution of iron nitrate hydrate (55.8 g/L) dissolved in distilled water were added into a precipitation reaction vessel, and the temperature was raised to 60°C while stirring at 400 rpm. In this case, the porous silica powder was input in an amount such that the iron content per 1 $m^2$ of its surface area was 13 mg. After the temperature of the solution reached 60°C, 144 g/L of aqueous ammonium carbonate solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.5 after the precipitation was completed. The produced precipitate was filtered to be separated from the solvent, washed with 2.5 L of distilled water, and completely dried at 110°C for 12 hours or more.

The content of iron in the Fe-$SiO_2$ powder obtained after drying was 60 wt%, and the content of iron was adjusted by adding copper and potassium. Copper and potassium were supported by adding an aqueous solution containing 55 g/L of copper nitrate hydrate and 55 g/L of potassium nitrate to the Fe-$SiO_2$ powder so that the weight ratio of iron/(copper + potassium) was 10. In this case, the copper and potassium were supported in the same weight. After the addition of copper and potassium, drying was performed at 110°C for 12 hours and calcination was performed at 400°C for 3 hours to manufacture a catalyst.

**Comparative Example 3**

200 mL of a solution of iron nitrate hydrate (55.8 g/L) dissolved in distilled water were added into a precipitation reaction vessel, and the temperature was raised to 60°C while stirring at 400 rpm. After the temperature of the solution reached 60°C, 120 g/L of aqueous sodium hydroxide solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.9 after the precipitation was completed. The produced precipitate was filtered to be separated from the solvent, washed with 2.5 L of distilled water, and completely dried at 110°C for 12 hours or more.

The iron content was adjusted by adding sodium to the Fe powder obtained after drying. Sodium was supported by adding an aqueous solution containing 110 g/L of sodium carbonate to the Fe powder so that the weight ratio of iron/sodium was 13. After the addition of sodium, drying was performed at 110°C for 12 hours and calcination was performed at 400°C for 3 hours to manufacture a catalyst.

Comparative Example 4

0.56 g of porous alumina ($\gamma$-$Al_2O_3$) powder having a specific surface area of 200 $m^2$/g and 200 mL of a solution of iron nitrate hydrate (55.8 g/L) dissolved in distilled water were added into a precipitation reaction vessel, and the temperature was raised to 60°C while stirring at 400 rpm. In this case, the porous alumina powder was input in an amount such that the iron content per 1 $m^2$ of its surface area was 100 mg. After the temperature of the solution reached 60°C, 180 g/L of aqueous ammonium carbonate solution was added at a rate of 2 mL/min using a syringe pump until the pH in the reactor became 7.0, and after the addition was completed, further stirring was performed at 400 rpm for 2 hours while maintaining 60°C to prepare a precipitate. The pH of the slurry was 7.1 after the precipitation was completed. The produced precipitate was filtered to be separated from the solvent, washed with 2.5 L of distilled water, and dried at 110°C for 12 hours (completely dried).

The content of iron in the $Fe-Al_2O_3$ powder obtained after drying was 67 wt%, and the content of iron was adjusted by adding copper and potassium. Copper and potassium were supported by adding an aqueous solution containing 50 g/L of copper nitrate hydrate and 50 g/L of potassium nitrate to the $Fe-Al_2O_3$ powder so that the weight ratio of iron/(copper + potassium) was 20. In this case, the copper and potassium were supported in the same weight. After the addition of copper and potassium, drying was performed at 110°C for 12 hours and calcination was performed at 400°C for 3 hours to manufacture a catalyst.

**Experimental Example 1**

The iron content (wt%), specific surface area, and iron content per catalyst surface area in each catalyst manufactured in the examples and comparative examples were measured, and the results are shown in Table 1 below.

(1) Iron content (wt%)

The iron content was measured using the ICP-OES method.

0.01 g of each catalyst was aliquoted, placed in a container containing 1 mL of hydrochloric acid along with hydrogen peroxide, and dissolved by heating with a hot plate. Thereafter, two drops of a 50 wt% aqueous hydrofluoric acid solution were added to the container by using a dropper, 0.1 mL of boric acid (57 g/L) was added 2 hours after the addition of the aqueous hydrofluoric acid solution, and then left to stand for 12 hours to completely dissolve the catalyst. After confirming that the catalyst was completely dissolved and the solution became transparent, the solution was diluted with 3rd ultrapure water to prepare an analysis sample solution. The iron content in each of the samples was measured by using iCAP PRO (ThermoFischer).

(2) Specific surface area ($m^2$/g)

The specific surface area of the catalyst was measured using a conventional nitrogen isothermal adsorption/-desorption method based on the Brunauer-Emmett-Teller (BET) theory. Each catalyst was pretreated at 150°C for 3 hours using BELPREP VAC II (MicrotracBEL), and then nitrogen isothermal adsorption/desorption results at 77K (-196°C) were obtained using BELSORP MAX (MicrotracBEL), and the specific surface area was calculated by applying the BET theory in the $P/P_0$ (Relative Pressure) range of 0.05-0.35.

(3) Iron content per surface area (mg/$m^2$)

The iron content (mg) per 1 $m^2$ of surface area was calculated from the iron content and specific surface area of the catalyst using Equation 4 below:

Iron content per surface area (mg/$m^2$)=[Iron content (wt%)/specific surface area ($m^2$/g)]$\times$1000 mg/g          [Equation 4]

[Table 1]

| Division | Fe content (wt%) | Specific surface area ($m^2$/g) | Iron content per catalyst surface area (mg/$m^2$) |
|---|---|---|---|
| Example 1 | 57.2 | 63 | 9.0 |
| Example 2 | 57.3 | 54 | 10.6 |
| Example 3 | 53.1 | 97 | 5.5 |
| Example 4 | 64.7 | 52 | 12.4 |
| Example 5 | 63.9 | 55 | 11.6 |
| Comparative Example 1 | 30.2 | 249 | 1.2 |
| Comparative Example 2 | 57.0 | 193 | 3.0 |
| Comparative Example 3 | 68.1 | 35 | 19.4 |

(continued)

| Division | Fe content (wt%) | Specific surface area (m²/g) | Iron content per catalyst surface area (mg/m²) |
|---|---|---|---|
| Comparative Example 4 | 64.8 | 42 | 15.5 |

As shown in Table 1 above, the catalysts manufactured in Examples 1 to 5 were confirmed to have iron content, specific surface area, and iron content per catalyst surface area all within the specific ranges suggested by the present invention. On the other hand, Comparative Examples 1 to 4 did not satisfy all or two of the characteristics of iron content, specific surface area, and iron content per catalyst surface area.

Through this, it was confirmed that the iron content, specific surface area, and iron content per catalyst surface area adjusted within the specific ranges suggested by the present invention are characteristics that can be obtained by the preparing method suggested by the present invention, which controls the ratio of the oxide-based support to iron when supporting iron on the oxide-based support, or controls the promoter metal at a weight ratio measured relative to iron.

## Experimental Example 2

Direct hydrogenation of carbon dioxide was performed using the catalysts manufactured in the examples and comparative examples to confirm the catalytic activity, and the results are shown in Table 2 below.

0.5 g of each catalyst was loaded into a fixed-bed tubular reactor (Stainless steel, diameter 3/8 inch), and before starting the reaction, the catalyst was activated by reduction treatment for 8 hours under a carbon monoxide atmosphere (>99.9 volume%) at 400°C. Thereafter, the raw material gas ($H_2$:$CO_2$:$N_2$=72:24:4 volume ratio) flowed at a flow rate of 75 mL/min, and the reaction temperature was adjusted to 340°C and the reaction pressure to 20 bar. The reaction was performed while maintaining the same flow rate of the raw material gas, the molar ratio of hydrogen to carbon dioxide in the raw material gas was set to 3, and nitrogen was added as an internal standard substance. During the total reaction period of 48 hours, the gas phase reactants and products were analyzed using gas chromatography (GC), and the liquid phase products and the incidental water were recovered from the cooling trap. The components of the liquid phase products were analyzed using a separate GC. The flow rate analysis before and after the reaction was measured through real-time measurement and the change in concentration of nitrogen, which is an internal standard. Based on the results measured using GC, the carbon dioxide ($CO_2$) conversion rate, turnover frequency (TOF), and C5+ selectivity (%) were calculated.

[Table 2]

| Division | Iron content per surface area (mg/m²) | $CO_2$ Conversion rate (%) | TOF (h⁻¹) | C5+ selectivity (%) |
|---|---|---|---|---|
| Example 1 | 9.0 | 44 | 4.0 | 47 |
| Example 2 | 10.6 | 45 | 4.1 | 54 |
| Example 3 | 5.5 | 45 | 4.4 | 40 |
| Example 4 | 12.4 | 46 | 3.7 | 54 |
| Example 5 | 11.6 | 47 | 3.8 | 50 |
| Comparative Example 1 | 1.2 | 12 | 2.1 | 4 |
| Comparative Example 2 | 3.0 | 46 | 4.1 | 32 |
| Comparative Example 3 | 19.4 | 42 | 3.2 | 55 |
| Comparative Example 4 | 15.5 | 42 | 3.3 | 54 |

As shown in Table 2 above, it was confirmed that the carbon dioxide ($CO_2$) conversion rate, turnover frequency (TOF), and C5+ selectivity (%) of Examples 1 to 5 were all superior to those of Comparative Examples 1 to 4. Specifically, Examples 1 to 5 showed a remarkable increase in the C5+ selectivity (%) by 10 times or more compared to Comparative Example 1, and also showed a significant increase in the carbon dioxide ($CO_2$) conversion rate and turnover frequency (TOF) by about 4 times and about 2 times, respectively. In addition, Examples 1 to 5 showed the same level of the carbon dioxide ($CO_2$) conversion rate and turnover frequency (TOF), but showed an improvement of about 1.25 times to about 1.72 times in the C5+ selectivity (%), compared to Comparative Example 2.

In addition, Examples 1 to 5 showed improved carbon dioxide ($CO_2$) conversion rates of about 5% to about 12%, and at the same time showed a significant improvement of about 6% to about 30% in turnover frequency (TOF), while showing an equivalent or slightly improved C5+ selectivity (%), compared to Comparative Examples 3 and 4.

Here, Examples 1 to 5 had an iron content per 1 $m^2$ of catalyst surface area of 5 mg to 15.0 mg, while the catalysts of Comparative Examples 1 and 2 had iron contents per 1 $m^2$ of surface area of 1.2 mg and 3.0 mg, respectively, which was very low in the density of the catalyst surface Fe site, which is the reaction active site, compared to Examples 1 to 5. In the case of Comparative Examples 3 and 4, the iron content per 1 $m^2$ of the catalyst surface area was 19.4 mg and 15.5 mg, respectively, but the specific surface area was 35 $m^2$/g and 42 $m^2$/g, respectively, and thus the available catalyst surface area was half that of Example 1.

From the above results, it was confirmed that the catalyst satisfying the iron content, specific surface area, and iron content per catalyst surface area defined in the present invention had the effect of simultaneously improving the carbon dioxide ($CO_2$) conversion rate, turnover frequency (TOF), and C5+ selectivity (%).

**Claims**

1. A metal oxide-based catalyst, comprising:

    an oxide-based carrier; and catalyst particles including iron supported on the carrier,
    wherein the content of iron per 1 $m^2$ of surface area of the catalyst is 5 mg to 15 mg.

2. The catalyst according to claim 1, wherein the iron is included in an amount of 50 wt% or more and 65 wt% or less based on 100 wt% of the total catalyst.

3. The catalyst according to claim 1, wherein the specific surface area of the catalyst is 50 $m^2$/g to 100 $m^2$/g.

4. The catalyst according to claim 1, wherein the oxide-based carrier is at least one selected from the group consisting of $SiO_2$, $Al_2O_3$, and zeolite.

5. The catalyst according to claim 1, wherein the iron is in a hematite phase.

6. The catalyst according to claim 1, wherein the iron is activated to at least one selected from the group consisting of magnetite, iron carbide, and metallic iron (Fe).

7. The catalyst according to claim 1, wherein the catalyst particles further include a promoter metal,
wherein the promoter metal is at least one selected from the group consisting of copper, potassium, and sodium.

8. The catalyst according to claim 1, wherein the catalyst particles further include at least one promoter metal selected from the group consisting of copper, potassium, and sodium,
wherein the promoter metal is included in an amount of 0.1 to 20 parts by weight based on 100 parts by weight of the catalyst.

9. The catalyst according to claim 1, which is a catalyst for direct hydrogenation of carbon dioxide.

10. A method for preparing a catalyst, the method including the steps of:

    preparing iron oxide-based catalyst particles (S1);
    supporting a promoter metal on the catalyst particles (S2); and
    drying and calcining (S3),
    wherein the step (S1) comprises the steps of: preparing a first solution by dissolving an iron precursor in a solvent to have an iron weight concentration of 20 g/L to 100 g/L, and introducing and dispersing an oxide-based carrier into the first solution to prepare a second solution (S1-1);
    raising the temperature of the second solution to 40°C to 90°C while stirring the second solution, and adding an aqueous precipitant solution to prepare a precipitate including iron oxide-based catalyst particles (S1-2); and
    washing, filtering, and then drying the precipitate (S1-3),
    wherein in step (S1-1), the oxide-based carrier is introduced in an amount such that the iron content per 1 $m^2$ of the oxide-based carrier surface area is 10 mg to 150 mg, and
    wherein in step (S2), the promoter metal is supported in an amount such that the weight ratio of iron/promoter metal is 5 to 15.

11. The method for preparing a catalyst according to claim 10, wherein in step (S1-2) above, the aqueous precipitant solution contains the precipitant at a weight concentration of 50 g/L to 250 g/L, wherein the precipitant is at least one selected from the group consisting of sodium carbonate, ammonium carbonate, ammonia water, and sodium hydroxide.

12. The method for preparing a catalyst according to claim 10, wherein in step (S1-2) above, the aqueous precipitant solution is added until the pH in the reactor becomes 7 to 8.

13. The method for preparing a catalyst according to claim 10, wherein the iron precursor is at least one selected from the group consisting of iron nitrate, sulfate, acetate, chloride, bromide, iodide, and hydrates thereof.

14. The method for preparing a catalyst according to claim 10, wherein in step (S1-2) above, the stirring is performed at a speed of 100 rpm to 1000 rpm.

15. The method for preparing a catalyst according to claim 10, wherein in step (S1-2) above, after the addition of the aqueous precipitant solution is completed, further stirring is performed for 0.5 to 24 hours.

16. The method for preparing a catalyst according to claim 10, wherein in step (S2) above, the promoter metal is at least one selected from the group consisting of copper, potassium, and sodium.

17. The method for preparing a catalyst according to claim 10, wherein step (S2) above is performed by an incipient wetness impregnation method.

18. The method for preparing a catalyst according to claim 10, wherein in steps (S1-3) and (S3) above, the drying is performed at 100°C to 200°C for 5 to 24 hours, respectively.

19. The method for preparing a catalyst according to claim 10, wherein in step (S3) above, the calcination is performed by heat treatment at a temperature of 200°C to 600°C for 1 to 12 hours.

20. A method for producing a hydrocarbon compound, the method including a step of direct hydrogenation of carbon dioxide in the presence of the catalyst according to claim 1.

## INTERNATIONAL SEARCH REPORT

| | International application No. |
|---|---|
| | **PCT/KR2024/013606** |

### A. CLASSIFICATION OF SUBJECT MATTER

**B01J 23/745**(2006.01)i; **B01J 35/61**(2024.01)i; **B01J 35/30**(2024.01)i; **B01J 37/03**(2006.01)i; **C07C 1/12**(2006.01)i

According to International Patent Classification (IPC) or to both national classification and IPC

### B. FIELDS SEARCHED

Minimum documentation searched (classification system followed by classification symbols)

B01J 23/745(2006.01); B01J 21/04(2006.01); B01J 23/72(2006.01); B01J 23/755(2006.01); B01J 35/00(2006.01); C01B 32/50(2017.01); C07C 1/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 이산화탄소(carbon dioxide), 수소화(hydrogenation), 촉매(catalyst), 비표면적 (surface area), 담체(support), 조촉매(cocatalyst)

### C. DOCUMENTS CONSIDERED TO BE RELEVANT

| Category* | Citation of document, with indication, where appropriate, of the relevant passages | Relevant to claim No. |
|---|---|---|
| Y | LOPEZ LUNA, M. et al. Role of the Oxide Support on the Structural and Chemical Evolution of Fe Catalysts during the Hydrogenation of CO2. ACS Catalysis. 2021, vol. 11, no. 10, pp. 6175-6185. See abstract; and pages 6176-6182. | 1-20 |
| Y | CN 1495148 A (HYDROCARBAN TECHNOLOGY CO.) 12 May 2004 (2004-05-12) See claims 1-3. | 1-20 |
| Y | KR 10-2020-0082911 A (HANWHA SOLUTIONS CORPORATION) 08 July 2020 (2020-07-08) See claim 1; and paragraphs [0098]-[0100]. | 10-19 |
| A | KR 10-2023-0037841 A (KOREA RESEARCH INSTITUTE OF CHEMICAL TECHNOLOGY) 17 March 2023 (2023-03-17) See claims 1-9. | 1-20 |
| A | KR 10-2023-0040742 A (KOREA RESEARCH INSTITUTE OF CHEMICAL TECHNOLOGY) 23 March 2023 (2023-03-23) See claims 1-6. | 1-20 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **17 December 2024** | **17 December 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/013606**

| Patent document cited in search report | | | Publication date (day/month/year) | Patent family member(s) | | | Publication date (day/month/year) |
|---|---|---|---|---|---|---|---|
| CN | 1495148 | A | 12 May 2004 | CN | 100358846 | C | 02 January 2008 |
| KR | 10-2020-0082911 | A | 08 July 2020 | CN | 113164925 | A | 23 July 2021 |
| | | | | CN | 113164925 | B | 31 October 2023 |
| | | | | EP | 3907001 | A1 | 10 November 2021 |
| | | | | EP | 3907001 | A4 | 12 October 2022 |
| | | | | JP | 2022-513180 | A | 07 February 2022 |
| | | | | JP | 2023-113704 | A | 16 August 2023 |
| | | | | JP | 7352631 | B2 | 28 September 2023 |
| | | | | KR | 10-2311346 | B1 | 08 October 2021 |
| | | | | SG | 11202106362 | QA | 29 July 2021 |
| | | | | TW | 202027856 | A | 01 August 2020 |
| | | | | TW | I726493 | B | 01 May 2021 |
| | | | | US | 11878286 | B2 | 23 January 2024 |
| | | | | US | 2022-0023842 | A1 | 27 January 2022 |
| | | | | WO | 2020-141705 | A1 | 09 July 2020 |
| KR | 10-2023-0037841 | A | 17 March 2023 | KR | 10-2571531 | B1 | 29 August 2023 |
| KR | 10-2023-0040742 | A | 23 March 2023 | KR | 10-2572062 | B1 | 29 August 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230119552
- KR 101405090 B1
- KR 20140602 B1